(19) Europäisches Patentamt
European Patent Office
Office européen des brevets

(11) Publication number: **0 461 401 A1**

(12) **EUROPEAN PATENT APPLICATION**

(21) Application number: **91107659.4**

(51) Int. Cl.5: **C07D 213/803**

(22) Date of filing: **11.05.91**

(30) Priority: **15.06.90 US 538863**
**25.04.91 US 689001**

(43) Date of publication of application:
**18.12.91 Bulletin 91/51**

(84) Designated Contracting States:
**AT BE CH DE DK ES FR GB GR IT LI LU NL SE**

(71) Applicant: **AMERICAN CYANAMID COMPANY**
**1937 West Main Street P.O. Box 60**
**Stamford Connecticut 06904-0060(US)**

(72) Inventor: **Cevasco, Albert Anthony**
**21 Kingswood Drive**
**Belle Mead, New Jersey 08502(US)**
Inventor: **Chiarello, George Anello**
**44 Doe Drive**
**Yardville, New Jersey 08620(US)**
Inventor: **Rieker, William Frederick**
**7-06 Pheasant Hollow Drive**
**Plainsboro, New Jersey 08536(US)**
Inventor: **Doehner, Robert F.**
**1 Berkshire Drive**
**East Windsor, New Jersey 08520(US)**

(74) Representative: **Wächtershäuser, Günter, Dr.**
**Tal 29**
**W-8000 München 2(DE)**

(54) **Process for the preparation of dialkyl, pyridine-2,3-dicarboxylates and derivatives thereof from dialkyl dichloromaleate.**

(57) There is provided a process for the preparation of substituted and unsubstituted-2,3-pyridinedicarboxylate compounds by reacting a dialkyl dichloromaleate with a source of ammonia and an appropriately substituted $\alpha,\beta$-unsaturated aldehyde or ketone.

EP 0 461 401 A1

Pyridine-2,3-dicarboxylates are useful intermediates in the preparation of highly effective herbicidal 2-(2-imidazolin-2-yl)nicotinic acids, esters and salts. Imidazolinyl nicotinates and derivatives thereof are highly effective herbicides at low rates of application and demonstrate selective control of noxious weeds in the presence of important agronomic crops while exhibiting exceptionally low mammalian toxicity.

It is an object of this invention to provide a method for the preparation of substituted and unsubstituted-2,3-pyridinedicarboxylates utilizing dialkyl dichloromaleate, an ammonia source and an appropriately substituted-α,β-unsaturated aldehyde or ketone.

The invention herein described relates to a novel method for the preparation of substituted and unsubstituted 2,3-pyridinecarboxylates of formula I

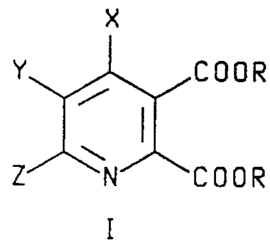

I

wherein R is $C_1$-$C_6$alkyl; X and Z are independently hydrogen, halogen, $C_1$-$C_4$alkyl, $C_1$-$C_4$alkoxy or $C_2$-$C_5$alkenyl and Y is hydrogen, halogen, $C_1$-$C_6$alkyl optionally substituted with one to three halogens, hydroxy groups, $C_1$-$C_4$alkoxy groups or $C_1$-$C_4$alkylthio groups, $C_1$-$C_4$alkoxycarbonyl, aminocarbonyl, phenyl, substituted phenyl, phenoxy, substituted phenoxy, phenylthio or substituted phenylthio. Compounds of formula I are useful as intermediates in the preparation of herbicidal 2-(2-imidazolin-2-yl)nicotinates. Among the methods of preparation of said herbicidal nicotinates is that described in U.S. Patent 4,758,667 and illustrated below.

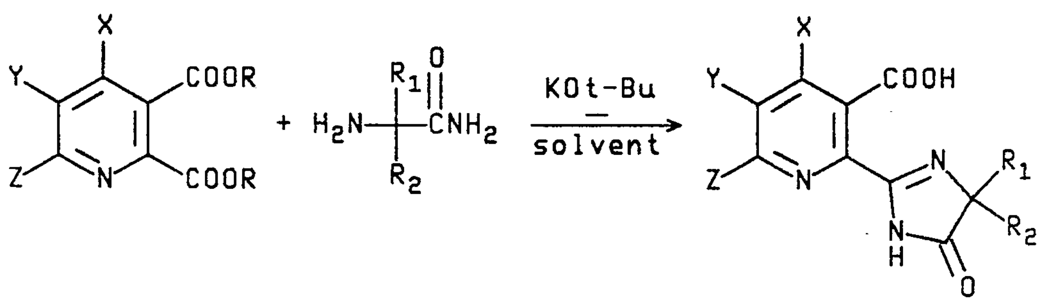


wherein $R_1$ is hydrogen or $C_1$-$C_4$alkyl; $R_2$ is hydrogen, halogen, $C_1$-$C_4$alkyl, $C_3$-$C_6$cycloalkyl or $R_1$ and $R_2$ may be taken together to form a $C_3$-$C_6$cycloalkyl optionally substituted with methyl and X, Y and Z are as described for formula I.

It has now been found that pyridine-2,3-dicarboxylates of formula I may be efficiently and effectively prepared from dialkyl dichloromaleates of formula II by reacting said maleate with an ammonia source in the presence of a suitable solvent such as a lover alkylalcohol to form an enamine intermediate and reacting said intermediate with at least one molar equivalent of an α,β-unsaturated aldehyde or ketone of formula III wherein X, Y and Z are as described above for formula I in the presence of an acid such as acetic acid. The processs is shown in Flow Diagram I.

FLOW DIAGRAM I

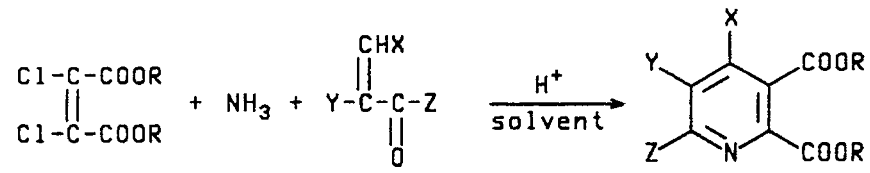

II III I

Ammonia sources may include organic ammonium salts such as ammonium acetate, ammonium propionate and the like, inorganic ammonium salts such as ammonium sulfate, ammonium carbonate, ammonium carbamate, ammonium sulfamate and the like, quaternary ammonium salts such as tetraalkylammonium halides, tetraalkylammonium sulfates and the like. Of course, ammonia itself may also be used as the ammonia source in the process of the invention.

Among the solvents suitable for use in the inventive process are polar solvents such as alcohols, nitriles such as acetonitrile, carboxylic acid amides such as N,N-dimethylformamide, N-methylpyrrolidone, sulfoxides such as dimethylsulfoxide, sulfones and the like.

Among the $\alpha,\beta$-unsaturated aldehydes or ketones of formula III which may be used in the process of the invention are acrolein, methacrolein, ethacrolein, crotonaldehyde, methyl vinyl ketone, $\alpha$-n-butylacrolein, 4-methyl-2-hexanal, $\alpha$-methoxymethacrolein, $\alpha$-chloromethacrolein, $\alpha$-trifluoromethacrolein, cinnamaldehyde, $\alpha$-ethoxyacrolein, methyl $\alpha$-formylacrylate, $\alpha$-(2-cyanoethyl)acrolein and the like.

Thus, pyridine-2,3-dicarboxylates containing substituents in the 4,5 and 6 positions may be conveniently prepared by admixing a formula II dialkyl dichloromaleate with an ammonia source in a suitable solvent, optionally heating said mixture until reaction is essentially complete, optionally filtering said reaction mixture, adding to the filtrate or to the unfiltered reaction mixture an acid such as a mineral acid, sulfuric acid, phosphoric acid, an organic acid such as acetic acid or propionic acid and the like to obtain a pH of at least about 4 and adding at least one molar equivalent of $\alpha,\beta$-unsaturated aldehyde or ketone of formula III, optionally heating the resulting reaction mixture until formation of the formula I pyridinedicarboxylate is complete. The reaction product may be isolated using standard purification techniques such as fractional distillation, recrystallization, extraction, liquid chromatography and the like.

The rate of formation of the enamine intermediate and the formula I pyridinedicarboxylate is temperature dependent, thus, reaction time may be effectively diminished by heating the reaction mixtures at temperatures of about 45°C or greater.

In one embodiment of the invention, the enamine intermediate may be prepared and isolated prior to reaction with the $\alpha,\beta$-unsaturated aldehyde or ketone of formula III and subsequently reacted with the formula III compound to give a pyridine-2,3-dicarboxylate of formula I. For example, dialkyl chlorooxalacetate may be reacted with at least one molar equivalent of ammonium sulfamate in the presence of a polar solvent at an elevated temperature to give the desired enamine compound of formula IV. The reaction is shown in flow diagram II

FLOW DIAGRAM II

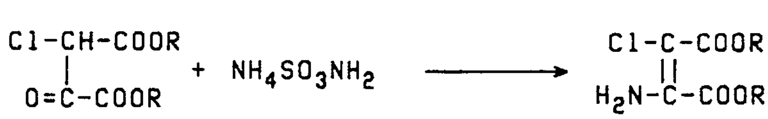

IV

wherein R is $C_1$-$C_6$alkyl.

The enamine compound of formula IV may be reacted with at least one molar equivalent of an $\alpha,\beta$-unsaturated aldehyde or ketone of formula III in the presence of a solvent and optionally in the presence of an acid to give the desired pyridinedicarboxylate of formula I. The reaction is illustrated in flow diagram III.

## FLOW DIAGRAM III

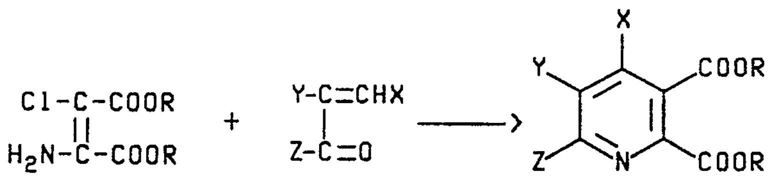

IV III I

Solvents suitable for use are as described hereinabove for flow diagram I. Reaction time may be effectively diminished by heating the reaction mixture at temperatures of about 45°C or greater.

In order to facilitate a further understanding of the invention, the following examples are presented primarily for the purpose of illustrating certain more specific details thereof and the invention is not to be deemed limited thereby. The terms $^1$HNMR and IR designate proton nuclear magnetic resonance and infrared, respectively. The term HPLC designates high performance liquid chromatography. Unless otherwise noted, all parts are parts by weight.

### EXAMPLE 1

**Preparation of diethyl 5-ethyl-2,3-pyridinedicarboxylate**

A solution of diethyl dichloromaleate (4.78 g, 0.02 mol) is treated with a total of (5.9 g, 0.35 mol) of anhydrous ammonia (1.2 g, 0.07 mol) and heated at 50°C until reaction is complete by thin layer chromatography. The reaction mixture is filtered and the filtrate is treated with acetic acid and 2-ethacrolein (4.61 g, 0.06 mol) and heated at 70°C until reaction is complete by chromatographic analysis. The reaction mixture is concentrated *in vacuo*, diluted with methylene chloride and filtered. The filtrate is fractionally distilled to give the title product as a tan oil, bp 151-152°C/2mm Hg.

### EXAMPLE 2

**Preparation of dialkyl (substituted)-2,3-pyridinedicarboxylate**

Using essentially the same procedure as described in Example 1 and substituting the appropriate α,β-unsaturated aldehyde and dialkyl dichloromaleate, the following pyridinedicarboxylates are obtained. The products are isolated by high performance liquid chromatography and identified by $^1$HNMR and IR spectographic analysis.

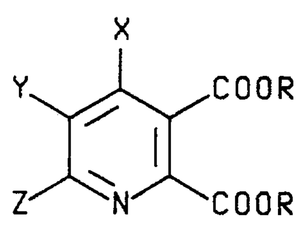

I

| X | Y | Z | R |
|---|---|---|---|
| H | $CH_3$ | H | $C_2H_5$ |
| H | $CH_2OCH_3$ | H | $CH_3$ |
| H | $CH_2Cl$ | H | $C_2H_5$ |
| H | H | H | $C_2H_5$ |

**EXAMPLE 3**

**Preparation of diethyl aminochlorobutenedioate**

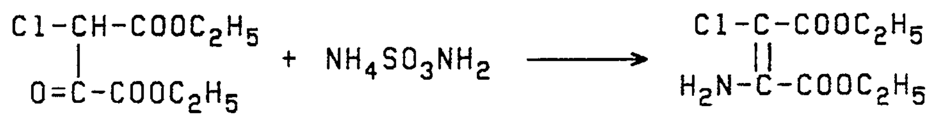

A mixture of diethyl chlorooxalacetate (22.5 g, 0.10 mole) and ammonium sulfamate (34.2 g, 0.30 mole) in absolute ethanol is heated at reflux temperature for 16 hours, cooled to room temperature and concentrated in vacuo to give a residue. The residue is partitioned between ether and water. The organic phase is dried ($MgSO_4$) and concentrated in vacuo to give an oil residue. After chromatography (silica gel and 4:1 hexanes:ether as eluent), the title compound is obtained as an off-white solid, mp 85-86°C.

**EXAMPLE 4**

**Preparation of diethyl 5-(methoxymethyl)-2,3-pyridinedicarboxylate**

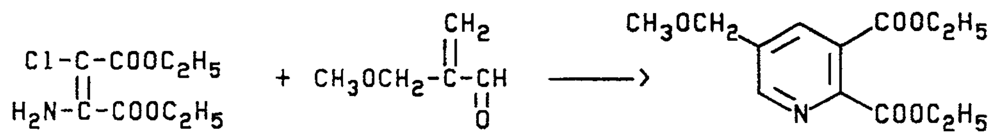

A solution of 2-(methoxymethyl)acrolein (2.65 g, 0.025 mole) in methanol and a solution of diethyl aminochlorobutenedioate (2.15 g, 0.010 mole) in methanol is added simultaneously to a mixture of methanol and acetic acid at 70°C over a period of 1 hour. The reaction mixture is heated at 70°C for 16 hours and after removal of methanol and acetic acid by vacuum distillation, the title product is obtained as a gum, identified by HPLC analysis.

**EXAMPLE 5**

**Preparation of diethyl 5-ethyl-2,3-pyridinedicarboxylate from diethyl aminochlorobutenedioate**

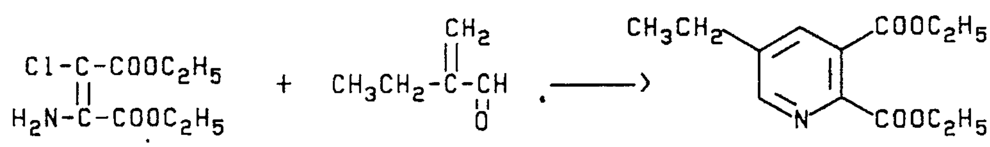

A mixture of diethyl aminochlorobutenedioate (1.1 g, 5.0 mmol) in absolute ethanol is heated at reflux temperature for 72 hours, cooled and concentrated in vacuo to give the title product as a gum, identified by $^1$HNMR analysis.

**Claims**

1. A process for the preparation of a compound characterized by formula I

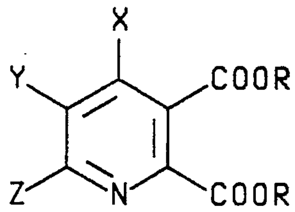

I

wherein R is $C_1$-$C_6$alkyl; X and Z are independently hydrogen, halogen, $C_1$-$C_4$alkyl, $C_1$-$C_4$alkoxy or $C_2$-$C_5$alkenyl and Y is hydrogen, halogen, $C_1$-$C_6$alkyl optionally substituted with one to three halogens, hydroxy groups, $C_1$-$C_4$alkoxy groups or $C_1$-$C_4$alkylthio groups, $C_1$-$C_4$alkoxycarbonyl, aminocarbonyl, phenylthio, substituted phenylthio, phenoxy, substituted phenoxy, phenyl or substituted phenyl which comprises reacting a dialkyl dichloromaleate of formula II

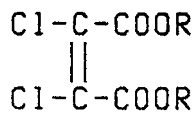

II

wherein R is $C_1$-$C_6$alkyl with an ammonia source in the presence of a solvent to form an enamine intermediate optionally filtering off insolubles and reacting said intermediate with an $\alpha,\beta$-unsaturated aldehyde or ketone of formula III

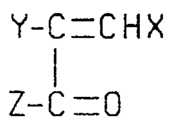

III

wherein X, Y and Z are as described above and an acid in the presence of the solvent to form the pyridinedicarboxylate compound of formula I.

2. The process according to claim 1 for the preparation of a compound characterized by formula I wherein X and Z are hydrogen and Y is hydrogen, halogen or $C_1$-$C_6$alkyl optionally substituted with one to three halogens, $C_1$-$C_4$alkoxy groups or $C_1$-$C_4$alkylthio groups.

3. The process according to claim 2 wherein the formula I compound prepared is selected from the group consisting of dialkyl pyridine-2,3-dicarboxylate, dialkyl 5-methyl-2,3-pyridinedicarboxylate, dialkyl 5-ethyl-2,3-pyridinedicarboxylate, dialkyl 5-(methoxymethyl)-2,3-pyridinedicarboxylate and dialkyl 5-(chloromethyl)-2,3-pyridinedicarboxylate.

4. The process according to claim 3 wherein the formula I compound prepared is selected from the group consisting of diethyl pyridine-2,3-dicarboxylate, diethyl 5-methyl-2,3-pyridinedicarboxylate, diethyl 5-ethyl-2,3-pyridinedicarboxylate, diethyl 5-(methoxymethyl)-2,3-pyridinedicarboxylate and diethyl 5-(chloromethyl)-2,3-pyridinedicarboxylate.

5. A pyridinedicarboxylate compound of formula I prepared by the process according to claim 1.

6. A process for the preparation of a compound characterized by formula I

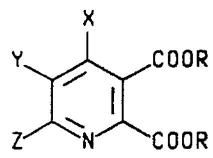

I

wherein R is $C_1$-$C_6$alkyl; X and Z are independently hydrogen, halogen, $C_1$-$C_4$alkyl, $C_1$-$C_4$alkoxy or $C_2$-$C_5$alkenyl and Y is hydrogen, halogen, $C_1$-$C_6$alkyl optionally substituted with one to three halogen, hydroxy, $C_1$-$C_4$alkoxy or $C_1$-$C_4$alkylthio groups, $C_1$-$C_4$alkoxycarbonyl, aminocarbonyl, phenylthio, substituted phenylthio, phenoxy, substituted phenoxy, phenyl or substituted phenyl which comprises reacting a dialkyl aminochlorobutenedioate of formula IV

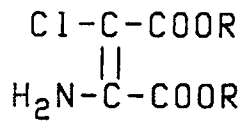

IV

wherein R is $C_1$-$C_6$alkyl with at least one molar equivalent of an $\alpha,\beta$-unsaturated aldehyde or ketone of formula III

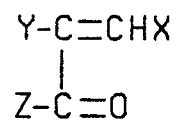

III

wherein X, Y and Z are as described above in the presence of a solvent and optionally in the presence of an acid.

7. The process according to claim 6 wherein the formula I compound prepared is selected from the group consisting of dialkyl pyridinedicarboxylate, dialkyl 5-ethyl-2,3-pyridinedicarboxylate, dialkyl 5-methyl-2,3-pyridine dicarboxylate, 5-(methoxymethyl)-2,3-pyridinedicarboxylate and 5-(chloromethyl)-2,3-pyridinedicarboxylate.

8. A pyridinedicarboxylate compound of formula I prepared by the process according to claim 6.

9. A dialkyl aminochlorobutenedioate compound of formula IV.

10. The compound according to claim 9, diethyl aminochlorobutenedioate.

European Patent Office

# EUROPEAN SEARCH REPORT

Application Number

**EP 91 10 7659**

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | EP-A-0 371 499 (WACKER-CHEMIE GMBH)<br>* the whole document *<br>- - - | 1,9 | C 07 D 213/803 |
| A | EP-A-0 308 084 (IMPERIAL CHEMICAL INDUSTRIES PLC)<br>* page 3 *<br>- - - | 1 | |
| A | EP-A-0 220 518 (AMERICAN CYNAMID COMPANY)<br>* pages 4 - 6 *<br>- - - - - | 1 | |
| | | | TECHNICAL FIELDS SEARCHED (Int. Cl.5) |
| | | | C 07 D 213/00 |

The present search report has been drawn up for all claims

| Place of search | Date of completion of search | Examiner |
|---|---|---|
| Berlin | 24 September 91 | KYRIAKAKOU G |

CATEGORY OF CITED DOCUMENTS

X: particularly relevant if taken alone
Y: particularly relevant if combined with another document of the same catagory
A: technological background
O: non-written disclosure
P: intermediate document
T: theory or principle underlying the invention

E: earlier patent document, but published on, or after the filing date
D: document cited in the application
L: document cited for other reasons

&: member of the same patent family, corresponding document